# EUROPEAN PATENT SPECIFICATION

(11) **EP 2 292 278 B1**
(45) Date of publication and mention of the grant of the patent: **24.04.2013**
(21) Application number: 10010950.3
(22) Date of filing: 12.11.2002
(51) Int. Cl.: A61L 27/36, A61F 2/00, A61L 27/38

(54) **Augmentation of organ function**
Verbesserung der Funktion von Organen
Augmentation de la fonction d'un organe

(30) Priority: 16.11.2001 US 331500 P
(43) Date of publication of application: 09.03.2011
(62) Divisional of application: 06022636.2
(73) Proprietor: Children's Medical Center Corporation, Boston, MA 02115 (US)
(72) Inventor: Atala, Anthony, Winston Salem, NC 27104-1927 (US)
(74) Representative: Strehl Schübel-Hopf & Partner

(56) References cited:
- WO-A-00/66036
- WO-A-01/48153
- WO-A-01/49210
- WO-A-88/03785
- WO-A-98/09582

## Description

### Reference to Related Applications

This application claims the priority of U.S. Provisional Application Serial No. 60/331,500, filed November 16, 2001. This application is also a continuation-in-part of U.S. Patent Application Serial No. 09/474,525, filed December 29, 1999.

### Background Of The Invention

The technical field of this invention is augmentation of organ function by implantation of cultured cell populations. Typically, a significant percentage of any organ function may be lost before a patient suffers complete organ failure. For example, as much as 90 to 95 percent of kidney function can be lost before kidney failure becomes apparent. This demonstrates the tremendous capacity for rapid self-renewal (Cuppage et al., (1969) Lab. Invest. 21:449-459; Humes et al., (1989) J. Clin. Invest. 84:1757-1761; and Witzgall et al. (1994) J. Clin. Invest. 93:2175-2188). This regenerative capacity allows the kidney to recover normal function within days or weeks.

Disruption of normal kidney function and capacity may arise due to a plethora of mechanisms such as infections, circulatory failure (shock), vascular blockage, glomerulonephritis, obstruction to urine flow, kidney failure associated with trauma, sepsis, postoperative complications, or medication, particularly antibiotics. Most of these reasons lead to reduced kidney function capacity.

Treatment of some of these ailments typically involves dialysis, which removes the waste products and chemicals from the blood system or transplantation. Dialysis poses a significant inconvenience to most patients. Usually treatment regimes involve lengthy time periods during which the patient is attached to the dialysis unit. The dialysis procedure is also repeated multiple times during a week. In many cases, the patient experiences side effects, such as muscle cramps and hypotension associated with the rapid change in the patient's body fluid.

With kidney transplantation the main risk is kidney rejection, even with a good histocompatibility match. Immunosuppressive drugs such as cyclosporin and FK506 are usually given to the patient to prevent rejection. However, these immunosuppressive drugs have a narrow therapeutic window between adequate immunosuppression and toxicity. Prolonged immunosuppression can weaken immune systems, which can lead to a threat of infections developing. In some instances, even immunosuppression is not enough to prevent kidney rejection.

In an attempt to avoid these problems various methods have been reported in which the patients own kidney cells have been cultured *in vitro.* For example, U.S. Pat. No. 5,429,938 issued to Humes, describes a method of reconstructing renal tubules using cultured kidney cells. The reconstructed renal tubules can be implanted into the patient.

Naughton *et al.* disclosed a three-dimensional tissue culture system in which stromal cells are laid over a polymer,support system (*see* U.S. 5,863,531) and parenchyma cells are cultured on the stromal matrix. Vacanti *et al.* have also disclosed methods for culturing cells in a three-dimensional matrix made of a biodegradable polymer. The above methods rely on shaping the support structure into the desired configuration of the entire organ and such that this artificial organ can be implanted into the body cavity as a replacement. However, there are many circumstances where the entire organ does not need to be replaced because only a portion of the organ is damaged.

Accordingly, a need exists for better methods and compositions for augmenting organ function in ways that do not require growth or replacement of the entire organ. A need exists for a using smaller, simpler support structures that mimic the structures of the native organ.

WO-A-98/09582 discloses a prosthetic kidney made by seeding kidney cells onto a porous membrane.

### Summary of the Invention

The present invention is defined by the kidney function augment construct specified in claim 1. The dependent claims relate to preferred embodiments. Disclosed are compositions for augmenting organ functions using small-scale matrix implants generated by seeding tissue-specific or undifferentiated cells onto a matrix materials (e.g., a wafer, sponge, or hydrogel). The seeded matrix composition can then be cultured *in vitro* to form a three-dimensional biomatrix in which the cells have grown to produce a tissue layer that is capable of developing into a neomophic organ augmenting structure. Once implanted, the three-dimensional biomatrix develops and proliferates at one or more target site in the organ to augment organ function at the site(s).

The invention is based, in part, on the discovery that seeded mini-matrices will sustain active proliferation of additional cell populations. This may be due, in part,-to the increased surface area of the matrix structure which permits a prolonged period of active proliferation of new cells. The prolonged proliferation enables the cells to develop into an neomorphic organ augmenting structure, which itself can develop into an organ augmenting unit, or is able to provide support for the growth and development of other cell populations which develop into the organ augmenting structure. In addition, the matrix allows for a spatial distribution which mimics the conditions *in vivo,* thus allowing for the formation of a microenvironment that is conducive for cellular maturation and migration. This provides the correct spatial distances that enable cell-cell interaction to occur. The growth of cells in the presence of this matrix may be further enhanced by adding proteins, glycoproteins, glycosaminoglycans and a cellular matrix.

In one illustrative example useful to understand the invention artificial organ constructs are disclosed for augmenting function of an organ comprising: a three-dimensional biomatrix formed by perfusing a matrix material with at least one population of cultured cells, such that the cells attach to the matrix material and produce a tissue layer capable of augmenting organ function e.g., to augment an organ such as the heart, kidney, liver, pancreas, spleen, bladder, ureter or urethra.

The construct can be formed by seeding cells onto a mini-matrix material such as decellularized tissue, a hydrogel or a synthetic or natural polymer. Preferably, the matrix is a "mini-matrix," the greatest dimension of which is less than about 50 millimeters. In one preferred embodiment, the matrix is a substantial flat structure having a ratio of its greatest dimension to its thickness of greater than 5:1.

In another example, the augmenting organ structure is a kidney function augmenting structure comprising: a three-dimensional biomatrix formed by perfusing a matrix material with a population of renal cells, such that the renal cells attach to the matrix and produce a tissue layer that differentiates into a nephron structure, or a part of a nephron structure, thereby augmenting kidney function.

In yet another example, the matrix has been initially perfused with a population of endothelial cells, such that the endothelial cells attach to the matrix material to produce an endothelial tissue layer comprising a vascular system, followed by seeding with a second population of cells, such that the second cell population attaches to the endothelial tissue layer comprising the vascular system and differentiates to augment organ function.

The invention is drawn to augmenting organ function without replacing the entire organ, or reconstructing the entire organ. For example, to augment kidney function, a small biopsy can be taken from the kidney and the renal cells then expanded *in vitro.* After cell sorting to remove damaged cells, the normal cells can be placed on a matrix (e.g., EGA wafer, sponge, hydrogel) and cultured. The matrix is then cultured until the cells produce a renal tissue layer that is capable of differentiating into a neomorphic organ structure to produce a biomatrix. The biomatrix is then implanted back into the patient, to desired locations within the kidney.

All kidney cells types can be isolated, i.e., proximal tubules, glomeruli, distil tubules and collecting ducts. The cells can be seeded separately or together. When the cells are seeded together_{;} it may be desirable to seed different types of cells sequentially onto the matrix, or in other instances, various cell types can be seeded together. The mixture of cells will regenerate into kidney tissue in a few weeks after implantation. In one embodiment of the invention, the kidney cells are placed on wafers of a polymer material, such as ethyl glycol acetate (EGA) or decellularized tissue. The wafers can be placed in any configuration suitable for implantation into a localized region, e.g., can be rolled, can be flat, etc. In one embodiment, the wafer can be placed in one location of the kidney. In another embodiment, a number of wafers can be placed at different locations in the organ. In one preferred embodiment, the matrices are miniaturized for greater ease of implantation. In many applications mini-matrices are desirable having sizes in which the greatest dimension is on the order of 50 millimeters or less, preferably 25 millimeters or less, and most preferably 10 millimeters or less. For example, polymeric wafers can have dimensions of about 2-5 mm, preferably about 2-3 mm. The wafers preferably are thin enough to allow vascularity to occur between the cells on the wafers and those of the surroundings. In one embodiment, the matrix can be treated with growth factors, e.g., VEGF.

During *in vitro* growth, the cells develop and produce a tissue layer which envelopes the matrix material. The tissue layer is capable of developing into a neomorphic organ augmenting structure and supports the growth and development of additional cultured cell populations. In one embodiment, the tissue layer can be derived form renal cells. In another embodiment, the tissue layer can be derived from endothelial cells that that develop to produce a primitive vascular system. This primitive vascular system can continue to grow and develop, and further support the growth of other parenchyma cells.

In the illustrative examples, the augmentation target is an organ selected from the group consisting of heart, kidney, liver, pancreas, spleen, bladder, ureter and urethra. In other illustrative examples, the augmentation target is a part of an organ selected from the group consisting of heart, kidney, liver, pancreas, spleen, bladder, ureter and urethra. In the preferred embodiment, the target organ is a kidney.

A method of treating a subject with an organ disorder comprises: implanting a biomatrix formed by seeding a matrix material with a population of cells, such that the cells attach to the matrix to produce proto-tissue comprising a primitive vascular system, capable of differentiating into a neomorphic organ structure; and monitoring the subject for a modulation in the organ disorder.

In another illustrative example, an artificial organ construct comprises: a matrix formed by seeding a matrix material with a population of cells, such that the cells attach to the matrix to produce tissue comprising a primitive vascular system, capable of differentiating into a neomorphic organ structure.

A method for reconstructing an artificial kidney construct comprises: seeding tissue-specific or Undifferentiated cells onto a matrix material, such as a wafer, sponge, or hydrogel), such that cells attach to the matrix; culturing the cells in and on the matrix until the cells produce a tissue structure; and implanting the seeded matrix at a target site for organ augmentation *in vivo.*

A method of treating a subject with a kidney disorder comprises: a matrix formed by seeding a matrix material with a population of cells, such that the cells attach to the matrix to produce tissue comprising a primitive vascular system, capable of differentiating into a neomorphic kidney structure; and monitoring the subject for a modulation in the kidney disorder.

In another illustrative example, an artificial kidney construct comprises: a matrix formed by seeding a matrix material with a population of cells, such that the cells attach to the matrix to produce proto-tissue comprising a primitive vascular system, capable of differentiating into a neomorphic organ structure.

### Brief Description Of Drawings

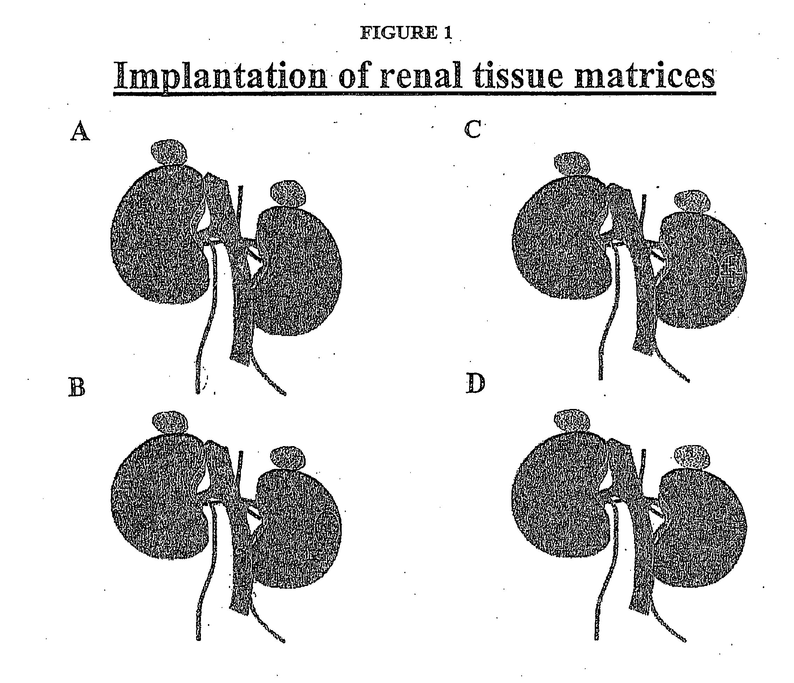

Fig.1 is a schematic diagram showing augmentation of a kidney using a biomatrix.

### Detailed Description

The practice of the present invention employs, unless otherwise indicated, conventional methods of microbiology, molecular biology and recombinant DNA techniques within the skill of the art. Such techniques are explained fully in the literature. (*See, e.g.,* Sambrook, et a/. Molecular Cloning: A Laboratory Manual (Current Edition); DNA Cloning: A Practical Approach, Vol. I & II(D. Glover, ed.); Oligonucleotide Synthesis (N. Gait, ed.; Current Edition); Nucleic Acid Hybridization (B. Hames & S. Higgins, eds., Current Edition); Transcription and Translation (B. Hames & S. Higgins, eds., Current Edition); CRC Handbook of Parvoviruses, Vol. I & II (P. Tijessen, ed.); Fundamental Virology, 2nd Edition, Vol. I & II (B. N. Fields and D. M. Knipe; eds.)). The invention also uses techniques described a tissue engineering literatures (see e.g., Principles of Tissue Engineering by Lanza et al (Current Edition). So that the invention may more readily be understood, certain terms are first defined:

The phrases "augmenting organ function" or "augmenting function of an organ" as used herein refers to increasing, enhancing, improving, the function of an organ that is operating at less than optimum capacity. The term is used to refer to a gain in function so that the organ is operating at a physiologically acceptable capacity for that subject. For example, the physiological acceptable capacity for an organ from a child, e.g., a kidney or heart, would be different from the physiological acceptable capacity of an adult, or an elderly patient. The entire organ, or part of the organ can be augmented. Preferably the augmentation results in an organ with the same physiological response as a native organ. In a preferred embodiment, an organ is augmented in capacity when it is functioning to at least at 10% of its natural capacity.

The phrases "three-dimensional biomatrix" or "augmenting construct" or "neomorphic organ augmenting structure" as used herein refers to a mini-matrix that has been perfused with the cells and cultured until the cells form a tissue layer. The tissue layer can be a single monolayer, or multiple layers. Tissue-specific cells refer to cells derived from the specific organ requiring augmentation, e.g., cells from a kidney organ for organ augmentation, and cells from a heart for heart organ augmentation. The cells in the three-dimensional biomatrix establish a "tissue-like" histology, can regenerate tissue-like architecture, and develop into primitive organoids with complex, multilayered structures that can eventually develop into the actual organ, or part of the organ. The three-dimensional biomatrix is an artificial organ, or part of an organ is the "functional equivalent" of the natural organ, i.e., behaves in the same, or similar manner as a natural organ, for example, the artificial kidney has the same functional characteristics as an *in vivo* kidney. For example, a kidney augmenting structure can be one that has a layer of tissue capable of developing into nephron structures, or part of a nephron structure. For kidney augmentation, the tissue specific cells can be an isolated population of cells selected from the group consisting of distil tubule cells, proximal tubule cells glomeruli cells, Bowman's capsule cells, and loop of Henlè cells. Alternatively, the tissue specific cells can be a mixed population of cells that includes distil tubule cells, proximal tubule cells, glomeruli cells, Bowman's capsule cells, and loop of Henlè cells. Various three-dimensional biomatrices that address specific diseases or disorders can be created. For example, the three-dimensional biomatrix can be specifically created to ameliorate disorders associated with the glomerulus by using an homogenous population of glomeruli cells that are used to perfuse the matrix material. Alternatively, the three-dimensional biomatrix can be a general construct created using a mixed population of renal cells.

When the three-dimensional biomatrix is brought into contact with a host tissue at a target site in the organ, it is able to grow and proliferate within the target site and replenish or augment the depleted activity of the organ at that site. The augmenting construct can be added at a single location in the organ. Alternatively, a plurality of augmenting constructs can be created and added to multiple sites in the organ.

The phrase "renal cells" as used herein refers to cells derived from any region of the kidney, such as cells from the distil tubule cells, proximal tubule cells, glomeruli cells, Bowman's capsule cells, or loop of Henlè cells. The term is used to refer a mixture of cells that includes all cells from the kidney. The term is also used to refer to an isolated sub-population of cells from a region of the nephron, e.g., a single population of only glomeruli cells. Cells form the kidney can be derived by taking a biopsy from the subject. Cell sorting techniques can be used to isolate healthy cells from diseased cells. Cell sorting techniques can also be used to isolated sub-populations of cells.

The term "nephron structure" as used herein refers the entire functional unit of the kidney that removes waste and excess substances from the blood to produce urine. Each of The million or so nephrons in each kidney are a tubule 1.2-2.2 inches (30-55 mm) long. At one end it is closed, expanded, and folded into a double-walled cuplike structure called the "Bowman's capsule", enclosing a cluster of capillaries called the "glomerulus". Fluid forced out of the blood through the capillary walls of the glomerulus into Bowman's capsule flows into the adjacent renal tubule, where water and nutrients are selectively reabsorbed from the fluid back into the blood, and electrolytes such as sodium and potassium are balanced in the loop of Henlé and proximal tubules. The final concentrated product is collected in the collecting duct as urine.

The term "part of a nephron structure" as used herein refers any section of the nephron. For example, a cell population can be sorted to produce an isolated population of only glomeruli cells. This isolated population of glomeruli cells can be used to seed a mini-matrix material and cultured to produce a three-dimensional mini-biomatrix with a glomeruli tissue layer that differentiates into a glomerulus. The same methodology can be applied to generate three-diniensional mini-biomatrices with specific cells derived from different regions of the nephron, that differentiate into the that part of the nephron, such as the distil tubule region. Other kidney disorders that are associated with a particular region of the nephron include those pathologies associated with the tubular cells.

The term "target site" as used herein refers to region in the organ that requires augmentation. The target site can be a single region in the organ, or can be multiple regions in the organ. The entire organ, or part of the organ can be augmented. Preferably the augmentation results in an organ with the same physiological response as a normal organ. The entire organ can be augmented by placing a plurality of biomatrices at suitable distances along the entire organ, e.g., along the entire longitudinal section of a kidney. Alternatively, part of the organ can be augmented by placing at least one biomatrix in one target site of the organ, e.g., the top of the kidneys.

The term "attach" or "attaches" as used herein refers to cells adhered directly to the matrix or to cells that are themselves attached to other cells.

The phrase "mini-matrix material" as used herein refers to a supportive framework that allows cells to attach. Preferably, the "mini-matrix," has a greatest dimension which is less than about 50 millimeters. In one preferred embodiment, the matrix is a substantial flat structure having a ratio of its greatest dimension to its thickness of greater than 5:1. The mini-matrix material is composed of any material and/or shape that allows cells to attach to it, or in it (or can be modified to allow cells to attach to it, or in it); and allows cells to grow into at least one monolayer or at least one tissue layer. Cultured populations of cells can then be grown on the matrix, or within the matrix. In one embodiment, the matrix material is a polymeric matrix which provides the desired interstitial distances required for cell-cell interaction. In another embodiment, the matrix material is a hydrogel composed of crosslinked polymer networks which are typically insoluble or poorly soluble in water, but can swell to an equilibrium size in the presence of excess water. Due to the unique properties of hydrogels and their potential applications in such areas as controlled drug delivery, various types of hydrogels have been synthesized and characterized.

The size of the mini-matrix material also varies according to the area of the organ being augmented. The size is typically smaller than the entire organ. Preferably, the volume of the matrix can range from about 1mm³ to the size of the organ. Most preferably, the size in volume is about 0.01mm³ to about 30mm³, more preferably, about 0.1mm³ to about 20mm³, even more preferably about 1mm³, 2mm³, 3mm³, 4mm³, 5mm³, 6mm³, 7mm³, 8mm³, 9mm³, and 10mm³ in volume. Elongate or flat matrices are preferably in many applications. Preferably, the length of greatest dimension of the matrix is greater than 0.2mm and less than 100mm, more preferably ranging from about 0.50mm to about 30mm. In a preferred embodiment, the shape of the mini-matrix is substantially flat and has a ratio of its greatest dimension to its thickness of greater than 5:1, more preferably greater than 10:1.

The shape and dimensions of the mini-matrix material is determined based on the organ being augmented, and the type of mini-matrix material being used to create the mini-biomatrix. For example, if a polymeric matrix is used for kidney augmentation, the dimension of the polymeric matrix can vary in terms of width and length of the polymeric matrix, for example the dimensions can be about 1mm width x 1mm length x 1mm height to about 10mm width x 20mm length x 1mm height. The skilled artisan will appreciate that the size and dimensions of the polymric matrix will be determined based on the area of the organ being augmented, as well as the actual organ being augmented.

Alternatively, if the matrix material is a hydrogel that is being used to augment a kidney, then the volume of the hydrogel can be determined based on the size of the area being-augmented in the kidney. For example, a volume of about 1mm³, 2mm³, 3mm³, 4mm³, 5mm³, 6mm³, 7mm³, 8mm³, 9mm³, and 10mm³ into which a population of cells cultured. In one embodiment, the hydrogel can be injected into one or more target sites in the organ. The volume of the hydrogel can be altered based on the organ and area of the organ being augmented. For example if the organ is a heart, and an area of infarction in the heart is being augmented, the volume of the hydrogel can range from a volume smaller than the size of the infarction to a volume that is the actual size of the infarction.

The term "biostructure" as used herein refers to parts of organs that have been decellularized by removing the entire cellular and tissue content from the part of the organ.

The term "decellularized" or "decellularization" as used herein refers to a biostructure (*e.g*., an organ, or part of an organ), from which the cellular and tissue content has been removed leaving behind an intact acellular infra-structure. Organs such as the kidney are composed of various specialized tissues. The specialized tissue structures of an organ, or parenchyma, provide the specific function associated with the organ. The supporting fibrous network of the organ is the stroma. Most organs have a stromal framework composed of unspecialized connecting tissue which supports the specialized tissue. The process of decellularization removes the specialized tissue, leaving behind the complex three-dimensional network of connective tissue. The connective tissue infra-structure is primarily composed of collagen. The decellularized structure provides a matrix material onto which different cell populations can be infused. Decellularized biostructures can be rigid, or semi-rigid, having an ability to alter their shapes. Examples of decellularized organs useful in the present invention include, but are not limited to, the heart, kidney, liver, pancreas; spleen, bladder, ureter and urethra.

The phrase "three-dimensional scaffold" as used herein refers to the residual infra-structure formed when a natural biostructure, *e.g*. an organ, is decellularized. This complex, three-dimensional, scaffold provides the supportive framework that allows cells to attach to it, and grow on it. Cultured populations of cells can then be grown on the three-dimensional scaffold, which provides the exact interstitial distances required for cell-cell interaction. This provides a reconstructed organ that resembles the native *in vivo* organ. This three-dimensional scaffold is perfused with a population of cultured endothelial cells which grow and develop to provide an endothelial tissue layer comprising a primitive vascular system that is capable of developing into a mature vascular system. The endothelial tissue layer and the primitive vascular system is also capable of supporting growth and development of at least one additional cultured cell population.

The term "primitive vascular system" as used herein refers to the early stages of development of a vascular system comprising blood vessels that supply blood to the tissue structures.

The term "subj ect," as used herein, refers to any living organism capable of eliciting an immune response. The term subject includes, but is not limited to, humans, nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

### I. Isolation and Culture of Cells

Cells can be isolated from a number of sources, for example, from biopsies, or autopsies, a stem cell population that is programmed to differentiate into the desired organ cells, a heterologous cell population that has been encapsulated to render it non-immunogenic, xenogenic cells, and allogenic cells. Also disclosed are methods which involve transfecting the cell population with factors such as growth factors which improve tissue formation.

The isolated cells are preferably allogenic, autologous cells, obtained by biopsy from the subject. For example, kidney cells can also be derived from the subject's dysfunctional kidney and cultured *in vitro.* The biopsy can be obtained using a biopsy needle, or a rapid action needle which makes the procedure quick and simple. The area for biopsy can be treated with local anaesthetic with a small amount of lidocaine injected subcutaneously. The small biopsy core of the organ, e.g., a kidney can then be expanded and cultured *in vitro,* as described by Atala, et al., (1992) J. Urol. 148, 658-62; Atala, et al. (1993) J. Urol. 150: 608-12. Cells from relatives or other donors of the same species can also be used with appropriate immunosuppression.

Methods for the isolation and culture of cells are discussed in Fauza et al. (1998) J. Ped: Surg. 33, 7-12 and Freshney" Culture of Animal Cells, A Manual of Basic Technique, 2d Ed., A.R. Liss, Inc., New York, 1987, Ch. 9, pp. 107-126. Cells may be isolated using techniques known to those skilled in the art. For example, the tissue or organ can be disaggregated mechanically and/or treated with digestive enzymes and/or chelating agents that weaken the connections between neighboring cells making it possible to disperse the tissue into a suspension of individual cells without appreciable cell breakage. Enzymatic dissociation can be accomplished by mincing the tissue and treating the minced tissue with any of a number of digestive enzymes either alone or in combination. These include but are not limited to trypsin, chymotrypsin, collagenase, elastase, and/or hyaluronidase, DNase, pronase and dispase. Alternatively, mechanical disruption can be used and this can be accomplished by a number of methods including, but not limited to, scraping the surface of the organ, the use of grinders, blenders, sieves, homogenizers, pressure cells, or insonicators. For a review of tissue disaggregation techniques, see Freshney, (1987), Culture of Animal Cells. A Manual of Basic Technique, 2d Ed., A. R. Liss, Inc., New York, Ch. 9, pp. 107-126.

Preferred cell types include, but are not limited to, kidney cells, endothelial cells, heart cells, liver cells, pancreatic cells, spleen cells, urothelial cells, mesenchymal cells, smooth or skeletal muscle cells, myocytes (muscle stem cells), fibroblasts; chondrocytes, adipocytes, fibromyoblasts, and ectodermal cells, including ductile and skin cells, hepotocytes, Islet cells, cells present in the intestine, and other parenchymal cells, osteoblasts and other cells forming bone or cartilage. In some cases, it may also be desirable to include nerve cells. Kidney cells are isolated. Kidney cells from all developmental stages, such as, fetal, neonatal, juvenile to adult may be used. In another preferred embodiment, endothelial cells are isolated.

Once the tissue has been reduced to a suspension of individual cells, the suspension can be fractionated into subpopulation from which the cells elements can be obtained. This also may be accomplished using standard techniques for cell separation including, but not limited to, cloning and selection of specific cell types, selective destruction of unwanted cells (negative selection), separation based upon differential cell agglutinability in the mixed population, freeze-thaw procedures, differential adherence properties of the cells in the mixed population, filtration, conventional and zonal centrifugation, centrifugal elutriation (counterstreaming centrifugation), unit gravity separation, countercurrent distribution, electrophoresis and fluorescence-activated cell sorting. For a review of clonal selection and cell separation techniques, see Freshney, (1987), Culture of Animal Cells. A Manual of Basic Techniques, 2d Ed., A. R. Liss, Inc., New York, Ch. 11 and 12, pp. 137-168. For example, kidney renal cells may be enriched by fluorescence-activated cell sortings. Also, different regions of the renal cells may be sorted into separate sub-populations. For example, separate sub-populations of glomeruli cells, bowman's capsule cells, distil tubule cells proximal tubule cells, loop of Henlè cells and collective duct cells.

Cell fractionation may also be desirable to sort healthy cells from diseased cells, for example, when the donor has diseases such as cancer or metastasis of tumors. A cell population may be sorted to separate malignant cells or other tumor cells from normal noncancerous cells. The normal noncancerous cells, isolated from one or more sorting techniques, may then be used for organ augmentation.

Isolated cells can be cultured *in vitro* to increase the number of cells available for coating the matrix material. The use of allogenic cells, and more preferably autologous cells, is preferred to prevent tissue rejection. However, if an immunological response does occur in the subject after implantation of the artificial organ, the subject maybe treated with immunosuppressive agents such as, cyclosporin or FK506, to reduce the likelihood of rejection. In certain embodiments, chimeric cells, or cells from a transgenic animal, can be coated onto the matrix material. Alternatively stem cells may be used.

Stem cells can also be used to generate the neomorphic organ augmenting structures of the invention. Stem cells can be derived from a human donor, e.g., pluripotent hematopoietic stem cells, adult somatic stem cells, and the like. The stem cells can be cultured in the presence of combinations ofpolypeptides, recombinant human growth and maturation promoting factors, such as cytokines, lymphokines, colony stimulating factors, mitogens, growth factors, and maturation factors, so as to differentiate into the desired cells type, e.g., renal cells, or cardiac cells. Method for stem cell differentiation into kidney and liver cells from adult bone marrow stem cells (BMSCs) are described for example by Forbes et al. (2002) Gene Ther 9:625-30. Multipotent stem cells from metanephric mesenchyme can generate at least three distinct cell types; glomerular, proximal and distal epithelia, i.e., differentiation into a single nephron segment (*See e.g.,* Herzlinger et al. (1992) Development 114:565-72).

The organ cells, e.g., kidney cells, could be transfected with specific genes prior to coating the matrix material. The neomorphic organ augmenting structure could carry genetic information required for the long term survival of the host or the organ being augmented.

Isolated cells can be normal or genetically engineered to provide additional or normal function. For example, cells can be transfected with compounds that reduce the programs of the disease at the target site in the organ. Cells may also be engineered to reduce or eliminate an immune response in the host. For example, the expression of cell surface antigens such as class I and class II histocompatibility antigens may be suppressed. This may allow the transplanted cells to have reduced chance of rejection by the host In addition, transfection could also be used for gene delivery. Methods for genetically engineering cells with retroviral vectors, polyethylene glycol, or other methods known to those skilled in the art can be used. These include using expression vectors which transport and express nucleic acid molecules in the cells (*See* Goeddel; Gene Expression Technology: Methods in Enzymology 185, Academic Press, San Diego, CA (1990).

The cells grown on the matrix may be genetically engineered to produce gene products beneficial to transplantation, e.g., anti-inflammatory factors, e.g., anti-GM-CSF, anti-TNF, anti-IL=1, and anti-IL-2. Alternatively, the endothelial cells maybe genetically engineered to "knock out" expression of native gene products that promote inflammation, e.g., GM-CSF, TNF, IL-1, IL-2, or "knock out" expression ofMHC in order to lower the risk of rejection. In addition, the cells maybe genetically engineered for use in gene therapy to adjust the level of gene activity in a patient to assist or improve the results of tissue transplantation.

Methods for genetically engineering cells with retroviral vectors, polyethylene glycol, or other methods known to those skilled in the art can be used. These include using expression vectors which transport and express nucleic acid molecules in the cells. (*See* Geoddel; Gene Expression Technology: Methods in Enzymology 185, Academic Press, San Diego, CA (1990).

Vector DNA is introduced into prokaryotic or eukaryotic cells via conventional transformation or transfection techniques. Suitable methods for transforming or transfecting host cells can be found in Sambrook et al. Molecudar Cloning: A Laboratory Manual, 2nd Edition, Cold Spring Harbor Laboratory press (1989), and other laboratory textbooks. The seeded cells can be engineered using a recombinant DNA construct containing the gene of interest that transforms or transfects the cells. The seeded matrix comprising the transfected cells expresses the active gene product, could be implanted into an individual who is deficient for that product. For example, genes that prevent or ameliorate symptoms of various types of vascular, genitourinary tract, hernia, gastrointestinal diseases, or kidney diseases may be underexpressed or down regulated under disease conditions. The level of gene activity may be increased by either increasing the level of gene product present or by increasing the level of the active gene product which is present in the biomatrix comprising the matrix material and tissue, e.g., endothelial tissue layer or renal tissue layer. The biomatrix culture can express the active target gene product can then be implanted into the patient who is deficient for that product.

The biomatrix cultures containing such genetically engineered cells can then implanted into the subject to allow for the amelioration of the symptoms of the disease. The gene expression may be under the control of a non-inducible (*i.e*., constitutive) or inducible promoter. The level of gene expression and the type of gene regulated can be controlled depending upon the treatment modality being followed for an individual patient.

### II. Matrix Materials

The composition of the present invention is created using matrix materials as the substrate onto which cells are deposited, and on which cells grown and adhere. It is important to recreate, in culture, the cellular microenvironment found *in vivo* for the particular organ targeted for augmentation. Retaining an infra-structure that is similar or the same as an *in vivo* organ creates the optimum environment for cell-cell interactions, development and differentiation of cell populations. The extent to which the cells and tissue layers are grown prior to use *in vivo* may vary depending on the type of organ being augmented.

Disclosed is a method of augmenting organ function using a matrix material that supports the maturation, development and differentiation, of additional cultured cells *in vitro* to form components of adult tissues analogous to their *in vivo* counterparts. The matrix allows optimum cell-cell interactions, thereby allowing a more natural formation of cellular phenotypes and a tissue microenvironment. The matrix also allows cells to continue to grow actively, proliferate and differentiate to produce a neomorphic organ augmenting structure that is also capable of supporting the growth, proliferation and differentiation of additional cultured cells populations.

Cells grown on the matrix materials, may grow in multiple layers, forming a cellular structure that resembles physiologic conditions found *in vivo*. The matrix can support the proliferation of different types of cells and the formation of a number of different tissues. Examples include, but are not limited to, kidney, heart, skin, liver, pancreas, adrenal and neurological tissue, as well as tissues of the gastrointestinal and genitourinary tracts, and the circulatory system.

The seeded matrices of the invention can be used in a variety of applications. For example, The matrices can be implanted into a subject. Implants, according to the invention, can be used to replace or augment existing tissue. For example, to treat a subject with a kidney disorder by augmenting the natural kidney. The subject can be monitored after implantation of the matrix implant, for amelioration of the kidney disorder.

Also there are compositions and methods of organ augmentation using a neomorphic organ augmenting structure with one population of cultured cells to produce a tissue layer from the single population. Alternatively, the neomorphic organ augmenting structure can comprise multiple layers derived from at least two different cell populations, e.g., a smooth muscle cell population, and a urotehlial cell population. In a preferred embodiment, the neomorphic organ augmenting structure comprises an endothelial layer with a primitive vascular system and at least one other tissue layer derived from parenchyma cells.

Once perfused onto the matrix material, the endothelial cells will proliferate and develop on the polymeric matrix to form an endothelial tissue layer. During *in vitro* culturing, the endothelial cells develop and differentiate to produce a primitive vascular system which is capable of developing into a mature vascular system, and is also capable of further development and is also capable of supporting the growth of parenchyma cells perfused into the matrix material. Importantly, because the polymeric matrix has an infra-structure that permits culture medium to reach the endothelial tissue layer and the parenchyma cells, the different cell populations continue to grow, divide, and remain functionally active. The parenchyma cells proliferate, and differentiate into neomorphic organ structures that have a morphology which resembles the analogous structure *in vivo.* The extent to which the endothelial cells and parenchyma cells are grown prior to use *in vivo* may vary depending on the type of organ being augmented. Organs that can be augmented include, but are not limited to, heart, kidney, liver, pancreas, spleen, bladder, ureter and urethra.

### (i) Polymeric matrices

The matrix material is a polymeric matrix. Examples of suitable polymers include, but are not limited to, collagen, poly(alpha esters) such as poly(lactic acid), poly(glycolic acid), polyorthoesters and polyanhydrides and their copolymers, cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyestercarbonate, polyether, polyetheretherketone, polyetherimide, polyetherketone, polyethersulfone, polyethylene, polyfluoroolefin, polylmide, polyolefin, polyoxadiazole, polyphenylene oxide, polyphenylene, sulfide, polypropylene, polystyrene, polysulfide, polysulfone, polytetrafluoroethylene, polythioether, polytriazole, polyurethane, polyvinylidene fluoride, regenerated cellulose, urea-formaldehyde, or copolymers or physical blends of these materials.

Polymers, such as polyglycolic acid, which is a suitable biocompatible structures for producing an organ augmenting structure. The biocompatible polymer may be shaped using methods such as, solvent casting, compression molding, filament drawing, meshing, leaching, weaving and coating.

In solvent casting, a solution of one or more polymers in an appropriate solvent, such as methylene chloride, is cast as a branching pattern relief structure. After solvent evaporation, a thin film is obtained.

In compression molding, a polymer is pressed at pressures up to 200 MPa (30,000 pounds per square inch) into an appropriate pattern. Filament drawing involves drawing from the molten polymer and meshing involves forming a mesh by compressing fibers into a felt-like material.

In leaching, a solution containing two materials is spread into a shape close to the final form of the organ. Next a solvent is used to dissolve away one of the components, resulting in pore formation (*See* Mikos, US 5,514,378).

In nucleation, thin films in the shape of the organ is exposed to radioactive fission products that create tracks of radiation damaged material. Next, the polycarbonate sheets are etched with acid or base, turning the tracks of radiation-damaged material into pores. Finally, a laser may be used to shape and bum individual holes through many materials to form an organ structure with uniform pore sizes.

The polymeric matrix can be fabricated to have a controlled pore structure that allows nutrients from the culture medium to reach the deposited cell population, but present cultured cells from migrating through the pores. *In vitro* cell attachment and cell viability can be assessed using scanning electron microscopy, histology and quantitative assessment with radioisotopes.

The polymeric matrix can be shaped into any number of desirable configurations to satisfy any number of overall system, geometry or space restrictions. The polymeric matrix can be shaped to different sizes to conform to the organs of different sized patients. The polymeric matrix may also be shaped to facilitate special needs of a patient, for example, a disabled patient, who may have a different abdominal cavity space may require a organ or part of an organ reconstructed to adapt to fit the space.

In illustrative examples, the polymeric matrix is used for the treatment of laminar structures in the body such as urethra, vas deferens, fallopian tubes, lacrimal ducts. In those applications the polymeric substrate can be shaped as a hollow tube.

The neomorphic organ augmenting structure; functioning to augment an organ, can be flat, tubular, or of complex geometry. The shape of the organ will be decided by its intended use. The artificial organ can be implanted to repair, augment, or replace diseased or damaged parts of organs. Flat sheets or wafers may be used. The flat sheets can be shaped to a desired shape and geometry to fit within the target site of an organ, e.g., rolled into a cylinder or tube. Tubular grafts may be used, for example, to replace cross sections of tubular organs such as esophagus, trachea, intestine, and fallopian tubes. These organs have a basic tubular shape with an outer surface and luminal surface.

A polymeric matrix can be permeated with a material, for example liquified copolymers (poly-DL-lactide co-glycolide 50:50 80 mg/ml methylene chloride) to alter its mechanical properties. This can be performed by coating one layer, or multiple layers until the desired mechanical properties are achieved. The size of the polymeric matrix is determined based on the extent of organ augmentation required. For example, the matrix can dimensions of about 1mm length x 1mm width x 1mm depth. The shape and size of the matrix depends on the region of the organ being augmented.

In one embodiment, the organ being augmented is a kidney, and the matrix can be a flat piece with dimensions of about 1 cm x 1 cm and a thickness less than about 1mm. Preferably, the length of greatest dimension of the matrix is greater than 0.2mm and less than 100mm, more preferably ranging from about 0.50mm to about 30mm. In a preferred embodiment, the shape of the mini-matrix is substantially flat and has a ratio of its greatest dimension to its thickness of greater than 5:1, more preferably greater than 10:1.

In another embodiment, the polymeric matrix can be rolled into a tube after the cells have been seeded to provide a larger volume of the organ structure. The size and shape of the polymeric matrix can be a disc, a wafer, a rolled wafer, a square, rectangle and the like. The configuration of the polymeric matrix is determined based on the area being augmented and, the organ being augmented. The size and shape of the polymeric matrix are selected such that the neomorphic organ augmenting structure that is produced has a ratio of its greatest dimension to its thickness of greater than 5:1, more preferably greater than 10:1.

In an illustrative example, the augmenting organ structure an be used to augment organs comprising multiple layers, e.g., a bladder. This can be performed by using one side of the polymeric matrix to create a tissue layer by coating one side of the polymeric matrix with a suspension of a first homogenous cell population, *e.g*., renal cells. The first homogenous cell suspension is incubated in culture medium until the cells develop and proliferate to produce a monolayer and cells of the monolayer attach to the polymeric matrix. Once the monolayer is established, the first homogenous cell suspension is deposited over the first monolayer, and the cells are cultured until they develop and proliferate to produce second monolayer of cells over the first monolayer, thereby producing a bilayer. The process is repeated until a polylayer comprising multiple layers of the first homogenous cell population is generated. The polylayer is cultured to produce a tissue layer with morphological and functional characteristics that allow it to differentiate into an organ augmenting structure.

In another embodiment, both sides of the polymeric matrix are used to create a polylayer of a homogenous cell population. This is performed by coating one side of the polymeric matrix with a suspension of a homogenous cell population, *e.g*., renal cells, and culturing the cells until they develop into a monolayer. Repeating the procedure on the opposite side of the polymeric matrix. The process is repeated on both sides of the polymeric matrix until a polylayer comprising multiple layers of the homogenous cell population is generated on both sides of the matrix. The polymeric matrix comprising the polylayers on both sides is cultured to produce a tissue layer with morphological and functional characteristics that allow it to differentiate into an organ augmenting structure. In yet another embodiment, both sides of the polymeric matrix can be used to create polylayers of different cell populations. This is performed by coating one side of the polymeric matrix with a suspension of a first homogenous cell population, *e.g*., endothelial cells, and culturing the cells until they develop into a monolayer. Repeating the procedure on the opposite side of the polymeric matrix with a different homogenous cell population, e.g., renal cells.

The organ being augmented is the kidney. The organ augmenting structure is created by using renal cells, or an isolated populations of distil tubule cells, proximal tubule cells, or glomeruli cells seeded in or on a matrix material. The kidney can be surgically opened along its longitudinal axis, and the neomorphic organ augmenting structure is placed in at least one target site in the kidney. In another embodiment, a plurality of neomorphic organ augmenting structures can be created and added at multiple target sites within the kidney. The number of neomorphic organ augmenting structure to be added depends of the extend of damage to the kidney. For example, if half of the upper half of the kidney is damaged, then about 1 to about 10 augmenting constructs in the form of wafers can be positioned equally along the upper half of the kidney. The number of augmenting constructs to be implanted also depends on the size of the wafers used to create them. If the wafers are of large dimension e.g., 1 cm x 1 cm x 1cm, then a fewer number of wafers are needed to augment the upper half of the kidney. Alternatively, if the wafers are small e.g., 1mm x 1mm x 1mm, then many of these are needed to augment the same upper half of the kidney.

### (ii) Hydrogels

In one embodiment, the matrix material is a hydrogel composed of crosslinked polymer networks which are typically insoluble or poorly soluble in water, but can swell to an equilibrium size in the presence of excess water. For example, the cells can be placed in a hydrogel and the hydrogel injected into desired locations within the organ. In one embodiment, the cells can be injected with collagen alone. In another embodiment, the cells can be injected with collagen and other-hydrogels. The hydrogel compositions can include, without limitation, for example, poly(esters), poly(hydroxy acids), poly(lactones), poly(amides), poly(ester-amides), poly(amino acids), poly(anhydrides), poly(ortho-esters), poly(carbonates), poly(phosphazines), poly(thioesters), polysaccharides and mixtures thereof. Furthermore, the compositions can also include, for example, a poly(hydroxy) acid including poly(alpha-hydroxy) acids and poly(beta-hydroxy) acids. Such poly(hydroxy) acids include, for example, polylactic acid, polyglycolic acid, polycaproic acid, polybutyric acid, polyvaleric acid, and copolymers and mixtures thereof. Due to the unique properties of hydrogels and their potential applications in such areas as controlled drug delivery, various types of hydrogels have been synthesized and characterized. Most of this work has focused on lightly crosslinked, homogeneous homopolymers and copolymers.

The bulk polymerization, i.e., polymerization in the absence of added solvent, of monomers to make a homogeneous hydrogel produces a glassy, transparent polymer matrix which is very hard. When immersed in water, the glassy matrix swells to become soft and flexible. Porous hydrogels are usually prepared by a solution polymerization techniques, which entails polymerizing monomers in a suitable solvent. The nature of a synthesized hydrogel, whether a compact gel or a loose polymer network, depends on the type of monomer, the amount of diluent in the monomer mixture, and the amount of crosslinking agent. As the amount of diluent (usually water) in the monomer mixture increases, the pore size also increases up to the micron range. Hydrogels with effective pore sizes in the 10-100 nm range and in the 100 nm- 10 micrometer range are termed "microporous" and "macroporous" hydrogels, respectively. The microporous and macroporous structures of hydrogels can be distinguished from those of non- hydrogel porous materials, such as porous polyurethane foams. In the plastic foam area, micro-and macro-pores are indicated as having pores less than 50 micrometers and pores in the 100-300 micrometer range, respectively. One of the reasons for this difference is that hydrogels with pores larger than 10 micrometers are uncommon, while porous plastics having pores-in the 100-300 micrometer range are very common.

Microporous and macroporous hydrogels are often called polymer "sponges." When a monomer, e.g., hydroxyethyl methacrylate (HEMA), is polymerized at an initial monomer concentration of 45 (w/w) % or higher in water, a hydrogel is produced with a porosity higher than the homogeneous hydrogels. The matrix materials of present invention encompass both conventional foam or sponge materials and the so-called "hydrogel sponges." For a further description of hydrogels, see U.S. Pat. No. 5,451,613 (issued to Smith et al.).

### (iii) Decellularized Parts of Biostructures

It is also disclosed herein that the neomorphic organ augmenting structure can be created using parts of a natural decellularized organ. Biostructures, or parts of organs can be decellularized by removing the entire cellular and tissue content from the organ. The decellularization process comprises a series of sequential extractions. One key feature of this extraction process is that harsh extraction that may disturb or destroy the complex infra-structure of the biostructure, be avoided. The first step involves removal of cellular debris and solubilization of the cell membrane. This is followed by solubilization of the nuclear cytoplasmic components an the nuclear components.

Preferably, the biostructure, e.g., part of an organ is decellularized by removing the cell membrane and cellular debris surrounding the part of the organ using gentle mechanical disruption methods. The gentle mechanical disruption methods must be sufficient to disrupt the cellular membrane. However, the process of decellularization should avoid damage or disturbance of the biostructure's complex infra-structure. Gentle mechanical disruption methods include scraping the surface of the organ part, agitating the organ part, or stirring the organ in a suitable volume of fluid, *e.g*., distilled water. In one preferred embodiment, the gentle mechanical disruption method includes stirring the organ part in a suitable volume of distilled water until the cell membrane is disrupted and the cellular debris has been removed from the organ.

After the cell membrane has been removed, the nuclear and cytoplasmic components of the biostructure are removed. This can be performed by solubilizing the cellular and nuclear components without disrupting the infra-structure. To solubilize the nuclear components, non-ionic detergents or surfactants may be used. Examples of non-ionic detergents or surfactants include, but are not limited to, the Triton series, available from Rohm and Haas of Philadelphia, Pa., which includes Triton X-100, Triton N-101, Triton X-114, Triton X-405, Triton X-705, and Triton DF-16, available commercially from many vendors; the Tween series, such as monolaurate (Tween 20), monopalmitate (Tween 40), monooleate (Tween 80), and polyoxethylene-23-lauryl ether (Brij. 35), polyoxyethylene ether W-1 (Polyox), and the like, sodium cholate, deoxycholates, CHAPS, saponin, n-Decyl β-D-glucopuranoside,n-heptyl β-D glucopyranoside, n-Octyl α -D-glucopyranoside and Nonidet P-40.

One skilled in the art will appreciate that a description of compounds belonging to the foregoing classifications, and vendors may be commercially obtained and may be found in "Chemical Classification, Emulsifiers and Detergents", McCutcheon's, Emulsifiers and Detergents, 1986, North American and International Editions, McCutcheon Division, MC Publishing Co., Glen Rock, N.J., U.S.A. and Judith Neugebauer, A Guide to the Properties and Uses of Detergents in Biology and Biochemistry, Calbiochem.R., Hoechst Celanese Corp., 1987. In one preferred embodiment, the non-ionic surfactant is the Triton. series, preferably, Triton X-100.

The concentration of the non-ionic detergent may be altered depending on the type ofbiostructure being decellularized. For example, for delicate tissues, *e.g*., blood vessels, the concentration of the detergent should be decreased. Preferred concentrations ranges non-ionic detergent can be from about 0.001 to about 2.0% (w/v). More preferably, about 0.05 to about 1.0% (w/v). Even more preferably, about, 0.1% (w/v) to about 0.8% (w/v). Preferred concentrations of these range from about 0.001 to about 0.2% (w/v), with about 0.05 to about 0.1 % (w/v) particular preferred.

The cytoskeletal component, comprising consisting of the dense cytoplasmic filament networks, intercellular complexes and apical microcellular structures, may be solubilized using alkaline solution, such as, ammonium hydroxide. Other alkaline solution consisting of ammonium salts or their derivatives may also be used to solubilize the cytoskeletal components. Examples of other suitable ammonium solutions include ammonium sulphate, ammonium acetate and ammonium hydroxide. In a preferred embodiment, ammonium hydroxide is used.

The concentration of the alkaline solutions, *e.g*., ammonium hydroxide, may be altered depending on the type ofbiostructure being decellularized. For example, for: delicate tissues, *e.g*., blood vessels, the concentration of the detergent should be decreased. Preferred concentrations ranges can be from about 0.001 to about 2.0% (w/v). More preferably, about 0.005 to about 0.1% (w/v). Even more preferably, about, 0.01% (w/v) to about 0.08% (w/v).

The decellularized, lyophilized structure may be stored at a suitable temperature until required for use. Prior to use, the decellularized structure can be equilibrated in suitable isotonic buffer or cell culture medium. Suitable buffers include, but are not limited to, phosphate buffered saline (PBS), saline, MOPS, HEPES, Hank's Balanced Salt Solution, and the like. Suitable cell culture medium includes, but is not limited to, RPMI 1640, Fisher's, Iscove's, McCoy's, Dulbecco's medium, and the like.

### III Cell Adhesion

In some embodiments, attachment of the cells to the matrix material is enhanced by coating the matrix material with-compounds such as basement membrane components, agar, agarose, gelatin, gum arabic, collagens types I, II, III, IV, and V, fibronectin, laminin, glycosaminoglycans, mixtures thereof, and other hydrophilic and peptide attachment materials known to those skilled in the art of cell culture. A preferred material for coating the matrix material is collagen.

In other embodiments, matrix materials can be treated with factors or drugs prior to implantation, before or after the matrix material is coated with cultured cells, *e.g*., to promote the formation of new tissue after implantation. Factors including drugs, can be incorporated into the matrix material or be provided in conjunction with the matrix material. Such factors will in general be selected according to the tissue or organ being reconstructed or augmented, to ensure that appropriate new tissue is formed in the engrafted organ or tissue (for examples of such additives for use in promoting bone healing, (*see, e.g.,* Kirker-Head, (1995) Vet. Surg. 24: 408-19). For example, when matrix materials are used to augment vascular tissue, vascular endothelial growth factor (VEGF), can be employed to promote the formation of new vascular tissue (*see, e.g.,* U.S. Patent No.5,654,273 issued to Gallo et al.). Other useful additives include antibacterial agents such as antibiotics.

### IV. Establishment of an Endothelial Tissue Layer

In one aspect, the invention pertains to using a cultured population of endothelial cells perfused on, or in the polymeric matrix material, or part of a decellularized organ, such that the endothelial cells grow and develop to produce a primitive vascular system. The endothelial cells may be derived from organs, such as, skin, liver, and pancreas, which can be obtained by biopsy (where appropriate) or upon autopsy. Endothelial cells can also be obtained from any appropriate cadaver organ. The endothelial cells can be expanded by culturing them *in vitro* to the desired cell density prior to infusion into the matrix material.

Endothelial cells may be readily isolated by disaggregating an appropriate organ, or part of an organ or tissue which is to serve as the source of the cells. This may be accomplished using techniques known to those skilled in the art. For example, the tissue or organ can be disaggregated mechanically and/or treated with digestive enzymes and/or chelating agents that weaken the connections between neighboring cells making it possible to disperse the tissue into a suspension of individual cells without appreciable cell breakage. Enzymatic dissociation can be accomplished by mincing the tissue and treating the minced tissue with any of a number of digestive enzymes either alone or in combination. These include, but are not limited to, trypsin, chymotrypsin, collagenase, elastase, and/or hyaluronidase, DNase, pronase, and dispase. Mechanical disruption can also be accomplished by a number of methods including, but not limited to, the use of grinders, blenders, sieves, homogenizers, pressure cells, or insonators to name but a few. (*See e.g.* Freshney, (1987) Culture of Animal Cells. A Manual of Basic Technique, 2d Ed., A. R. Liss, Inc., New York, Ch. 9, pp. 107-126.)

After reducing the tissue to a suspension of individual cells, the suspension can be fractionated into subpopulations from which the endothelial cells can be obtained. This also may be accomplished using standard techniques for cell separation including, but not limited to, cloning and selection of specific cell types, selective destruction of unwanted cells (negative selection), separation based upon differential cell agglutinability in the mixed population, freeze-thaw procedures, differential adherence properties of the cells in the mixed population, filtration, conventional and zonal centrifugation, centrifugal elutriation (counterstreaming centrifugation), unit gravity separation, countercurrent distribution, electrophoresis and fluorescence-activated cell sorting. (*See e.g.* Freshney, (1987) Culture of Animal Cells. A Manual of Basic Techniques, 2d Ed., A. R. Liss, Inc., New York, Ch. 11 and 12, pp. 137-168.)

The growth of cells in the matrix material, i.e. polymeric matrix, may be enhanced by adding, or coating the matrix material with proteins (*e.g*., collagens, elastic fibers, reticular fibers) glycoproteins, glycosaminoglycans (*e.g*., heparan sulfate, chondroitin-4-sulfate, chondroitin-6-sulfate, dermatan sulfate, keratin sulfate, etc.), a cellular matrix, and/or other materials.

After perfusion of the endothelial cells, the matrix material should be incubated in an appropriate nutrient medium. Many commercially available media such as RPMI 1640, Fisher's, Iscove's, McCoy's, Dulbecco's medium, and the like, may be suitable for use. The culture medium should also be changed periodically to remove the used media, depopulate released cells, and add fresh media. It is important to grow the endothelial cells to a stage where an endothelial tissue layer comprising a primitive vascular system has developed prior to perfusion of the endothelial tissue layer with the parenchyma cells.

### V. Perfusion of Parenchyma Cells onto Matrix Material Endothelial Layer

Once the endothelial tissue layer has reached the appropriate degree of growth and developed to produce a primitive vascular system, additional populations of cultured cells such as parenchymal cells can be perfused onto the endothelial tissue layer. Parenchyma cells perfused onto the endothelial tissue can be incubated to allow the cells to adhere to the endothelial tissue layer. The parenchyma cells can be cultured *in vitro* in culture medium to allow the cells to grow and develop until the cells resemble a morphology and structure similar to the that of the native tissue. Growth of parenchyma cells on the endothelial tissue layer results in the differentiation of parenchyma cells into the appropriate neomorphic organ augmenting structures.

Alternatively, after-perfusing the parenchyma cells, the matrix can be implanted *in vivo* without prior *in vitro* culturing of the parenchyma cells. The parenchyma cells chosen for perfusion will depend upon the organ being augmented. For example, augmentation of a kidney will involve infusing cultured endothelial cells into or onto a matrix material, which is cultured until they develop into endothelial tissue layer comprising a primitive vascular system. The endothelial tissue can then be perfused with cultured kidney cells and cultured *in vitro* until the kidney cells begin to differentiate to form nephron structures.

The parenchyma cells may be obtained from cell suspensions prepared by disaggregating the desired tissue using standard techniques as described above. The cells may then be cultured *in vitro* to a desired density. After attaining the desired density, the cultured cells can be used to perfuse the matrix material with the endothelial tissue layer. The cells will proliferate, mature, and differentiate on the endothelial tissue layer. The choice of parenchyma cells will depend on the organ being augmented for example, when augmenting a kidney, the matrix material, e.g., a polymeric matrix and endothelial tissue layer is perfused with cultured kidney cells. When augmenting an liver, the polymeric matrix and endothelial tissue layer is perfused with cultured hepatocytes. When augmenting a pancreas, the polymeric matrix and endothelial tissue layer is perfused with cultured pancreatic endocrine cells. When augmenting a pancreas, the polymeric matrix and endothelial tissue layer is perfused with cultured pancreatic endocrine cells. When augmenting a heart, the polymeric matrix and endothelial tissue layer is perfused with cultured cardiac cells. For a review of methods which may be utilized to obtain parenchymal cells from various tissues, *see,* Freshney, (1987) Culture of Animal Cells. A Manual of Basic Technique, 2d Ed., A. R. Liss, Inc., New York, Ch. 20, pp. 257-288. Cells are cultured until they differentiate to produce neomorphic organ augmenting structures that resemble the morphology of the native *in vivo* tissue

Growth factors and regulatory factors can be added to the media to enhance, alter or modulate proliferation and cell maturation and differentiation in the cultures. The growth and activity of cells in culture can be affected by a variety of growth factors such as insulin, growth hormone, somatomedins, colony stimulating factors, erythropoietin, epidermal growth factor, hepatic erythropoietic factor (hepatopoietin), and liver-cell growth factor. Other factors which regulate proliferation and/or differentiation include prostaglandins, interleukins, and naturally-occurring chalones.

### VI. Creation of Three-Dimensional Biomatrices

In one aspect, the invention pertains to creating three-dimensional biomatrices, or organ augmenting structures/contructs. In one embodiment, the organ augmenting structures are created to address a specific disease or disorder that disrupts the function of the organ. For example, an organ augmenting structures can be a specific augmenting structure created to augment, and thereby ameliorate, glomerulopathies associated with abnormal glomeruli function (e.g., glomerulerulopathies, such as primary glomerulerulopathies associated with impaired glomeruli filtration (e.g., acute nephritic syndrome, rapidly progressive glomerulonephritis (RPGN), glomeruli sclerosis, nephrotic syndrome, asymptomatic abnormalities of the urinary sediment (hepaturia, proteinuria), and chornic glomerulonephritis), or secondary glomerulerulopathies, associated with systemic disease (e.g., diabetic nephropathy and immunologically mediated multisystem disease). The organ augmenting structures can be created using an isolated population of glomeruli cells that are grown to a tissue layer, and then the construct implanted to treat glomeruli sclerosis. In another example, the organ augmenting structures can be created using an isolated population of tubule cells to augment, and thereby ameliorate tubular disorder such as proximal tubule dysfunction andrenal tubular acidosis. This allows the methods and compositions of the invention to be used to specific disorders and pathologies associated with particular regions of the nephron. Proximal tubule dysfunction may manifest aselective reabsorption defects leading to hypokalemia, aminoaciduria, glycosuria, phosphaturia, uricosuria, or bicarbonaturia. Renal tubular acidosis (RTA) results due to a defect in the reabsorption of filtered HCO₃, the excretion ofH, or both. Renal tubular acidosis is characteristically associated with hyperchloremia and a normal glomerular function. RTA can be classified as Distal RTA (RTA-1), Proximal RTA (RTA-2) and hyperkalemic RTA (RTA-4). The last one is seen in many hyperkalemic states and the defect is characterized by the inability of the tubule to excrete enough NH₄ as a direct consequence of increased cellular stores of K.

It is also disclosed herein that the organ augmenting structures can be a general augmenting structure created using a mixture of cells isolated from the organ being augmented. For example, an neomorphic organ augmenting structures seeded with renal cells comprising a mixture of distil tubule cells, proximal tubule cells, loop of Henle cells, and glomeruli cells.

In an illustrative example, the augmenting construct can be used to augment heart function. In this example, the neomorphic organ augmentings structures can be created by seeding the matrix material with a population of myocardial cells.

In another example, the augmenting construct can be used to augment bladder function. In this example, the augmenting construct can be created by seeding the matrix material with an isolated population of urothelial cells, or a population of cells comprising a mixture of smooth muscle cells and urothelial cells.

The biomatrices comprise a matrix material that as been perfused with at least one population of cultured cells, and incubated such that the dispersed cell population until it forms a monolayer and further incubating the cultured cells until they form a polylayer made up of multiple monolayers of cells, and eventually a tissue layer, e.g., renal tissue layer, or an endothelial layer with a primitive vascular system.

The sustained active proliferation of tissue layer eventually leads to the tissue layer resembling the equivalent parenchyma tissue of an *in vivo* organ. This may be due, in part, by the method of producing the polylayers. Polylayers are produced by culturing a first homogenous cell population one layer at a time on the matrix material until the cells of each layer are actively proliferating. The polylayers are incubated until the cells develop and proliferate to resemble the structure and morphology of the equivalent parenchyma tissue of an *in vivo* organ.

Polylayers developed by the method described above therefore produce proteins, growth factors and regulatory factors necessary to support the long term proliferation of the homogenous cell population. After the first polylayer has been established, this provides the surface for producing the second polylayer. The second polylayer comprises a second homogenous cell population that is different from the first homogeneous cell population. The second polylayer is developed by culturing the second homogenous cell population one layer at a time until the cells of each layer are actively proliferating to produce a polylayer of cells, and eventually a tissue layer.

This tissue layer is capable of differentiating into a organ augmenting structure with further *in vitro* incubation, or *in vivo* incubation. The growth of cells in the tissue layer may be further enhanced by adding factors such as nutrients, growth factors, cytokines, extracellular matrix components, inducers of differentiation, products of secretion, immunomodulators, biologically-active compounds which enhance or allow growth of the cellular network or nerve fibers proteins, glycoproteins, and glycosaminoglycans.

The matrix material used to create the biomatrix is a polymeric matrix. The tissue layer can be created on one side of the polymeric matrix, or both sides of the polymeric matrix until a tissue layer with the morphology and histology that allows differentiation into a organ augmenting structure is produced. In another embodiment, the polymeric matrix is a hydrogel into which a cultured cell population has been mixed. The cells are incubated in the hydrogel until they form a tissue layer that can differentiate into a organ augmenting structure.

### VII Implantation Of The Three-Dimensional Biomatrix.

The three-dimensional biomatrix or organ augmenting structures can be implanted into an organ requiring augmentation using standard surgical procedures. These surgical procedures may vary according to the organ being augmented. For kidney implantation, it maybe desirable to implant a series of three-dimensional biomatrices into incisions formed along the avascular plane of the kidney, or the least vascular region of an organ. In other applications, the constructs of the invention can be introduced by less invasive procedures, e.g., via a cannula, needle, trocar or catheter-type instrument.

### VIII Uses

The described methods and composition can be used to augment organ function in a variety of organs.

### (i) Kidney

The invention pertains to compositions for augmenting kidney function. Since virtually all kidney disease can cause renal failure, the major focus of treatment in most cases is to preserve kidney function. A subject typically has more kidney functioning power than necessary and most kidney diseases do not cause noticeable problems or symptoms until 90 percent of renal function is lost. Accordingly, the compositions of the invention can be used to augment kidney function of a kidney in which at least about 2% function, preferably about 5% function, more preferably about 10% function, even more preferably about 15, 20, 25, 30, 35, 40, 45, 50, 55, 60, 65, 70, 75, 80, 85, 90% function. The compositions of the invention can be used to ameliorate the symptoms of both acute and chronic renal failure. Kidney diseases that can be augmented include, but are not limited to, (for example, glomerulerulopathies, such as primary glomerulerulopathies associated with impaired glomeruli filtration (e.g., acute nephritic syndrome, rapidly progressive glomerulonephritis (RPGN), glomeruli sclerosis, nephrotic syndrome, asymptomatic abnormalities of the urinary sediment (hepaturia, proteinuria), and chornic glomerulonephritis), or secondary glomerulerulopathies, associated with systemic disease (e.g., diabetic nephropathy and immunologically mediated multisystem disease). This allows the compositions of the invention to be used to specific disorders and pathologies associated with particular regions of the nephron. Proximal tubule dysfunction may manifest aselective reabsorption defects leading to hypokalemia, aminoaciduria, glycosuria, phosphaturia, uricosuria, or bicarbonaturia. Renal tubular acidosis (RTA) results due to a defect in the reabsorption of filtered HCO₃, the excretion ofH, or both. Renal tubular acidosis is characteristically associated with hyperchloremia and a normal glomerular function. RTA can be classified as Distal RTA (RTA-1), Proximal RTA (RTA-2) and hyperkalemic RTA (RTA-4). The last one is seen in many hyperkalemic states and the defect is characterized by the inability of the tubule to excrete enough NN₄ as a direct consequence of increased cellular stores of K.

### (ii) Heart Disease

In an illustrative example, the methods and compositions are used to augment heart function in a subject with a heart disease or disorder heart failure is one of the leading causes of morbidity and mortality in the United States. Heart failure can result from any condition that reduces the ability of the heart to pump blood. Most frequently, heart failure is caused by decreased contractility of the myocardium, resulting from reduced coronary blood flow. Many other factors may result in heart failure, including damage to the heart valves, vitamin deficiency, and primary cardiac muscle disease. (Guyton (1982) Human Physiology and Mechanisms of Disease, Third Edition, W. B. Saunders Co., Philadelphia, Pa., p. 205). Heart failure is commonly manifested in association with myocardial infarction. (Manual of Medical Therapeutics (1989) Twenty-Sixth Edition, Little, Brown & Co., Boston (W. C. Dunagan and M. L. Ridner, eds.), pp. 106-09).

Heart failure in humans begins with reduced myocardial contractility, which leads to reduced cardiac output. The methods and composition described above may be used to augment heart function. For example by creating an neomorphic organ augmentive structure an area of the heart that has been damaged or infarcted or ischaemia.

Heart diseases include, but are not limited to angina pectoris, myocardial infarction, and chronic ischemic heart disease.

### (iii) Urogential disorders

In another illustrative example, the methods and compositions are used to augment urogenital organ function in a subject with a urogenital organ disease or a disorder. Examples of urogenital disorders include, but are not limited to those associated with the bladder, urethra, and ureter.

### (iv) Spleen disorders

The spleen is a small organ located next to the stomach that is part of the lymphatic system, the spleen helps protect the body against infection and filters blood. Patients who have had their spleen removed are more susceptible to certain types of infection. Accordingly, methods and compositions may be used to ameliorate or control spleen disorders, for example by using cells recombinantly modified to express agents that control the disease or disorder. Example of spleen disorders include, but are not limited to, idiopathic purpura, Felty's syndrome, Hodgkin's disease, and immune-mediated destruction of the spleen.

Other embodiments and uses of the invention will be apparent to those skilled in the art from consideration of the specification and practice of the invention disclosed herein.

### EXAMPLES

### Example 1: Isolation of Kidney Cells

Small kidneys, for example, from one week old C7 black mice, were decapsulated, dissected, minced and suspended in Dulbecco's Modified Eagles's Medium (DMEM; Sigma, St. Louis, MO) containing 15mM Hepes, pH 7.4 and 0.5 µg/ml insulin, 1.0 mg/ml collagenase and 0.5 mg/ml dispase, a neutral protease from *Bacillus polymyxal* (Boehringer Mannheim, Indianapolis, IN).

Large kidneys, for example, swine kidneys, were arterially perfused at 37°C for 10 minutes with calcium free Eagles minimum essential medium within three hours of extraction. The kidneys were then perfused with 0.5 mg/ml collagenase (Type IV, Sigma, St. Louis, MO) in the same buffer supplemented with 1.5 mM MgCl₂ and 1.5 mM CaCl₂. The kidneys were then decapsulated, dissected, minced and suspended in Dulbecco's Modified Eagles's Medium (DMEM; Sigma, St. Louis, MO) containing 15 mM Hepes, pH 7.4 and 0.5 µg/ml insulin, 1.0 mg/ml collagenase and 0.5 mg/ml dispase, a neutral protease from *Bacillus polymyxal* (Boehringer Mannheim, Indianapolis, IN).

The kidney cell suspension, from either large or small kidneys, was gently agitated in a water bath for 30 minutes at 37°C. The cells and fragments were recovered by centrifugation at 50g for five minutes. The pellets were resuspended in DMEM containing 10% fetal bovine serum (Biowhittaker, Walkersville, Maryland) to stop proteolysis, and the turbid solution was passed through sterile 80 mesh nylon screens to eliminate large fragments. The cells were recovered by centrifugation and washed twice with calcium free Dulbecco's Modified Eagles's Medium.

### Example 2: In vitro Culturing of Kidney Cells.

### (i) Isolation of rat tail collagen

Tendon was stripped from rat tails and stored in 0.12 M acetic acid in deionized water in 50 ml tubes. After 16 hours at 4°C overnight.

Dialysis bags were pretreated to ensure a uniform pore size and removal of heavy metals. Briefly, the dialysis bag is submerged in a solution of 2% sodium bicarbonate and 0.05% EDTA and boiled for ten minutes. Multiple rinses of distilled water was used to remove the sodium bicarbonate and 0.05% EDTA.

The 0.12 M acetic acid solution comprising rat tendons was placed in treated dialysis bags and dialyzed for two or three days to remove acetic acid. The dialysis solution was changed every 3 to 4 hours.

### (ii) Coating tissue culture plates:

The culture flasks, 75 cm²,were coated with a solution containing about 30 µg/ml collagen (Vitrogen or rat tail collagen), about 10 µg/ml human fibronectin (Sigma, St. Louis, MO) and about 10 µg/ml bovine serum albumin (Sigma, St. Louis, MO) in a total volume of about 2 ml of supplemented medium by incubation at 37°C for 3 hours.

### (iii) Cell culture

Digested single suspended renal cells were plated on, a modified collagen matrix at a concentration of about 1 x 10⁶ cells/ml and grown in DMEM supplemented with about 10% fetal bovine serum, about 5 µg/ml bovine insulin, about 10 µg/ml transferrin, about 10 µg/ml sodium selenite, about 0.5 µM hydrocortisone, about 10 ng/ml prostaglandin E₂, about 100 units/ml penicillin G, about 100 µg/ml streptomycin (Sigma, St. Louis, MO) in a 5% CO₂ incubator at about 37°C

Confluent monolayers, were subcultured by treatment with about 0.05% trypsin, about 0.53 mM EDTA (Gibco BRL, Grand Island, NY) in calcium ion free phosphate buffer saline (PBS) (about 1.51 mM KH₂PO₄, about 155.17 mM NaCl, about 2.8 mM Na₂HPO.7H₂O). Cells may be cultured any time from the first passage by suspension in about 10% DMSO in culture medium for freezing and storage in liquid medium.

### Example 3: Isolation and Culturing of Endothelial Cells.

Endothelial cells, were isolated from a dissected vein. Perivenous heparin/papaverine solution (3 mg papaverine HCl diluted in 25 ml Hanks balanced salt solution (HBSS) containing 100 units of heparin (final conc. µg/ml)), was used to improve endothelial cell preservation. A proximal silk loop was placed around the vein and secured with a tie. A small venotomy was made proximal to the tie and the tip of vein cannula was inserted and secured in place with a second tie. A second small venotomy was made beyond the proximal tie and the vein was gently flushed with Medium 199/heparin solution Medium 199 (M-199) supplemented with 20% fetal bovine serum, ECGF (1 00mg/ml L-glutamine, heparin (Sigma, 17.5µ/ml) and antibiotic-antimycotic), to remove blood and blood clots. Approximately 1 ml of a collagenase solution (0.2% Worthington type I collagenase dissolved in 98 ml of M-199, 1 ml of FBS, 1 ml of PSF, at 37°C for 15-30 min, and filter sterilized), was used to flush through the dissected vein. The collagenase solution was also used to gently distend the vein and the distended vein was placed into 50 ml tube containing Hank's Balanced Salt Solution (HBSS). The tube containing the collagenase distended vein was incubated for 12 minutes at 37°C to digest the inner lining of the vein. After digestion, the contents of the vein, which contain the endothelial cells, were removed into a sterile 15 ml tube. The endothelial cell suspension was centrifuged at 125 x g for 10 minutes. Endothelial cells were resuspended in 2 ml of Dulbecco.'s Modified Eagle Media with 10% FBS and penicillin/streptoniycin (DMEM/10%FBS) and plated into a 24 well plate coated with 1% difcogelatin. The endothelial cells were incubated overnight at 37°C.

After overnight incubation, the cells were rinsed with HBSS and placed in 1ml of fresh DMEM/10%FBS. The media was changed 3 times a week. When cultures reached confluence (after 3-7 days), the confluent monolayers were subcultured by treatment with 0:05% trypsin, 0.53 mM EDTA, for 3-5 min until the cells dispersed. The dispersed cells were plated onto culture dishes coated with 0.1% difcogelatin at a 1:4 - I :6 split ratio. The endothelial cells were expanded until sufficient cell quantities were achieved. Cells were trypsinized, collected, washed and counted for seeding.

### Reference Example 4: Isolation and Culturing Of Urothelial and Smooth Muscle Cells

The harvested cells were cultured according to previously published protocols of Atala et al., (1993) J. Urol. 150: 608, Cilento et al., (1994) J. Urol. 152: 655, Fauza et al., (1998) J. Ped. Surg, 33, 7-12.

### a) Culturing urothelial cell populations

A bladder specimen was obtained and prepared for culturing. To minimize cellular injury, the specimen was sharply excised rather than cut with an elecrocautery. The serosal surface was marked with a suture to ensure there will be no ambiguity as to which side represented the urothelial surface.

The specimen was processed in laminar flow cell culture hood, using sterile instruments. Culture medium with Keratinocyte-SFM (GIBCO BRL (Cat. No. 17005), with Bovine Pituitary Extract (Cat. No. 13028, 25 mg/500 ml medium) and Recombinant Epidermal Growth Factor (Cat. No. 13029, 2.5 µg/500ml medium) as supplement was prepared. 10 ml of culture medium at 4°C, was placed in each of two 10 cm cell culture dishes, and 3.5 ml in a third dish. Blood was removed from the specimen by placing the specimen in the first dish and gently agitating it back and forth. The process was repeated in the second dish, and finally the specimen was transferred to the third dish. The urothelial surface was gently scraped with a No. 10 scalpel blade without cutting into the specimen. The urothelial cells were visible as tiny opaque material dispersing into the medium. The urothelial cell/medium suspension was aspirated and seeded into six wells of a 24-well cell culture plate with approximately 0.5 to 1 ml of medium to each well to give a total of 1 to 1.5 ml per well. The cells were incubated at 37°C with 5% CO₂.

The following day (Day 1 post harvesting), the medium was aspirated from the six wells and fresh medium applied. the cells were centrifuged at 1000 rpm for 4 minutes and the supernatant was removed. The cells were resuspended in 3 to 4.5 ml of fresh medium warmed to 37°C in a 24-well plate.

The culture medium was removed and PBS/EDTA (37°C, pH 7.2, 0.53mM EDTA (0.53ml of 0.5M EDTA, pH 8.0, in each 500 ml of PBS)), was added to each 24-well plate well, or 10 ml to each 10 cm dish. The cells were then passaged in two 10 cm dishes. Hereafter the cells were passaged whenever they reached 80 to 90% confluence, without allowing the cells to reach 100% confluence.

The cells were observed under a phase contrast microscope. When the cell-cell junctions were separated for the majority of the cells (approximately 5 to 15 minutes), the PBS/EDTA was removed and 300 µl Trypsin/EDTA (37°C, GIBCO BRL, Cat. No. 25300-054), was added to each 24-well plate well or, 7 ml to each 10 cm dish. The plate/dish was periodically agitated. When 80 to 90% of the cells detached from the plate and started to float (approximately 3 to 10 minutes), the action of the Trypsin was inhibited by adding 30 µl soy bean Trypsin inhibitor (GIBCO BRL, Cat. No. 17075-029, 294 mg of inhibitor to 20 ml PBS), to each 24-well place well or 700 µl to each 10 cm dish to stop the action of the EDTA. 0.5 ml culture medium was added to each 24-well plate well or 3 ml culture medium was added to each 10 cm dish. The PBS/EDTA and Trypsin/EDTA incubations were performed at room temperature, but were more effective if the plates were incubated at 37°C.

The cells were harvested by centrifugation at 1000 rpm for 4 minutes, and the supernatant removed. The cells were resuspended in 5ml culture medium, and the number of cells was determined using a hemocytometer. Cell viability was determined by the standard Trypan blue stain test. The optimal seeding density for a 100 mm culture plate was approximately 1 x 10⁶cells/plate. The desired number of cells was aliquoted into the dish and the volume of a medium was added to a total of approximately 10 ml/plate.

### b) Culturing bladder smooth muscle cells.

After removing the urothelial cell layer from the bladder specimen as described inabove, the remaining muscle was dissected into 2-3 mm muscle segments. Each muscle segment was spaced evenly onto a 100 mm cell culture dish. The muscle segments were dried and allowed to adhere to the dish (approximately 10 minutes). 20 ml ofDulbecco's Modified Eagle Media with 10% FCS was added to the dried muscle segments. The muscle segments were incubated for 5 days undisturbed at 37°C with 5% CO₂. The culture media was changed on the 6th day and any non-adherent segments were removed. The remaining segments were cultured for a total of 10 days, after which all the muscle segments were removed. The cells from the muscle segments that had adhered to the dish were incubated until small islands of cells appeared. These cells were trypsinized, counted and seeded into a T75 culture flask.

The cells were fed every 3 days depending on the cell density, and the cells were passaged when they reached 80-90% confluence.

### Reference Example 5: Isolation and Culturing Of Cardiac Cells

This example describes one method of culturing cardiac cells. Cardiac cells, e.g., from atrial tissue can be obtained from mammals. Atrial tissue can be obtained for example, from the right atrial appendages harvested from cardiovascular surgery patients undergoing procedures requiring heart-lung bypasses. The appendages can be removed and placed in ice-saline slush for rinsing. The tough epicardial covering can be removed using a scalpel to reduce the amount of connective tissue included in the cell harvest. The remaining atrial muscle can be minced into small (0.5-1.0 mm³) pieces and placed in cold Hank's Balanced Salt Solution (HBSS) without calcium or magnesium (Whittaker, Walkerville, Mass.). The minced atrial tissue can be digested in 0.14% collagenase solution (Worthington, Freehold, N.J.) at a concentration of 1.43 mg/ml. The pieces can be placed in 35 ml of this solution and digested in a shaker at 37°C at 125 RPM for one hour. The supernatant can be removed from the atrial tissue and centrifuged at 3500 RPM for 10 minutes at 37°C. Another 35 ml of collagenase solution can be placed with the minced tissue while the supernatant was spinning and the digestion continued for another hour. The supernatant collagenase solution can be removed and set aside for use in the third digestion. The cell pellet can be resuspended in 2 ml of Eagle's Minimal Essential Medium (EMEM) with Earle's Salts (Whittaker) containing 30% newborn calf serum (Whittaker) and 0.1% antibiotic solution-10,000 units/cc Penicillin G,10,000 µg/cc Streptomycin and 25 µg/cc Amphotericin B (Gibco, Grand Island, N.Y.). This process can be continued for a further digestions.

The various digestions can be pooled, and the cell concentration can be checked using a hemacytometer and adjusted to 1X 10⁵ cells/ml with EMEM. The cells can be plated on 35 mm gelatin coated dishes (Corning, Coming, N.Y.) and incubated at 37°C in 5% CO₂ atmosphere. Medium can be changed every three days for the first two weeks of growth, then every five to seven days thereafter. When the cultures spread out and approach confluence, they can be treated with trypsin and transferred to 60 mm gelatin coated dishes (Corning) in EMEM. When the cells approach confluence, they can be treated with trypsin and transferred to T-75 flasks (Corning) in MCDB 107(Sigma, Saint Louis, Mo.).

A portion of the cells grown in MCDB 107 can be plated on four chamber gelatin coated slide culture plates (Lab Tek, Naperville, Ill.). Control cells can be human umbilical endothelial cell and human skin fibroblast cultures (Beaumont Research Institute, Royal Oak, Mich.) that can be grown in M199 with 20% fetal bovine serum, 1% L-glutamine, 0.1% of 5 mg/ml insulin-5 mg/ml transferrin-5 µg/ml selenious acid (Collaborative Research Inc.), 0.6 ml heparin (0.015% in M199), 0.1% antibiotic-antimycotic solution (Gibco Laboratories: 10,000 units/ml sodium pennicilin G, 100,000 mcg/ml streptomycin sulfate and 25 mcg/ml amphotericin B), and 300 µg/ml of Endothelial Cell Growth Supplement (ECGS) from Biotechnology Research Inst., Rockville, Md. When control cultures and harvested cells spread out and approached confluence they can be rinsed with HBSS and fixed with 10% formalin for 10 minutes. The chambers can be removed and the cells remaining on the plates can be stained with immunoperoxidase stains for smooth muscle alpha-actin (Lipshaw, Detroit, Mich.), striated muscle specific myosin (Sigma, St. Louis, Mo.), myoglobin (Dako, Carpinteria, Calif.), factor VIII (Lipshaw, Detroit, Mich.), and atrial natriuretic factor peptide (Research and Diagnostic Antibodies, Berkeley, Calif.). The plates can then be examined using light microscopy.

A portion of cells growing in MCDB 107 can be plated on 96-well gelatin-coated plates (Coming). When they spread out and approach confluence they can be rinsed with HBSS and fixed with 2.5% glutaraldehyde, 0.2M cacodyiate buffer, pH 7.4 (Polysciences, Inc., Warrington, Pa.), post-fixed with 1% osmium tetroxide (Polysciences, Inc.), embedded in Epon LX-112 resin (Ladd's Research, Burlington, Va.), stained with 0.03% lead citrate (Eastman Kodak, Rochester, N.Y.) and saturated uranyl acetate (Pelco Co., Tustin, Calif) in 50% ethyl alcohol and then examined under transmission electron microscopy.

### Reference Example 6: Preparation of a Decellularized Organs, or Parts of Organs

The following method describes a process for removing the entire cellular content of an organ or tissue without destroying the complex three-dimensional infra-structure of the organ or tissue. A kidney, was surgically removed from a C7 black mouse using standard techniques for tissue removal. The kidney was placed in a flask containing a suitable volume of distilled water to cover the isolated kidney. A magnetic stir plate and magnetic stirrer were used to rotate the isolated kidney in the distilled water at a suitable speed for 24-48 hours at 4°C. This process removes the cellular debris and cell membrane surrounding the isolated kidney.

After this first removal step, the distilled water was replaced with a 0.05% ammonium hydroxide solution containing 0.5% Triton X-100. The kidney was rotated in this solution for 72 hours at 4°C using a magnetic stir plate and magnetic stirrer. This alkaline solution solubilized the nuclear and cytoplasmic components of the isolated kidney. The detergent Triton X-100, was used to remove the nuclear components of the kidney, while the ammonium hydroxide solution was used to lyse the cell membrane and cytoplasmic proteins of the isolated kidney.

The isolated kidney was then washed with distilled water for 24-48 hours at 4°C using a magnetic stir plate and magnetic stirrer. After this washing step, removal of cellular components from the isolated was confirmed by histological analysis of a small piece of the kidney. If necessary, the isolated kidney was again treated with the ammonium hydroxide solution containing Triton X-100 until the entire cellular content of the isolated kidney was removed. After removal of the solubilized components, a collagenous three-dimensional framework in the shape of the isolated kidney was produced.

This decellularized kidney was equilibrated with 1 x phosphate buffer solution (PBS) by rotating the decellularized kidney overnight at 4°C using a magnetic stir plate and magnetic stirrer. After equilibration, the decellularized kidney was lyophilized overnight under vacuum. The lyophilized kidney was sterilized for 72 hours using ethylene oxide gas. After sterilization, the decellularized kidney was either used immediately, or stored at 4°C or at room temperature until required. Stored organs were equilibrated in the tissue culture medium overnight at 4°C prior to seeding with cultured cells.

### Example 7: Preparation of a Kidney Augmenting Organ Structure

This example describes the preparation of wafers for implantation into one or more regions of an organ, e.g., a kidney. The size and configuration of the renal tissue matrix (wafer) for placing in the kidney parenchyma is determined. For example, a matrix about 1mm thick, that is about 2cm in length and width. Single suspended renal cells are seeded on kidney tissue matrix at a concentration of about cells 10 x 10⁶ cells cm³. The cells were allowed to attach onto the matrix wall for about 2 hours at 37°C. The matrix was then turned over to the opposite side and single suspended renal cells were seeded on kidney tissue matrix. The cells were allowed to attach onto the matrix wall for about 2 hours at 37°C. After incubation is completed, culture medium was slowly added to the flask to cover the entire renal matrix. Care was taken not to disturb the cells within the matrix. The matrix was incubated at 37°C in an incubator with CO₂. The culture medium was changed daily, or more frequently, depending on the level of lactic acid produced. On day 4 after the initial seeding, the cell-matrix system was placed in a rotating bioreactor system for additional 3 days in order to achieve uniform cell distribution and growth.

### Example 8: Implantation of the Kidney Augmenting Organ Structure

The kidney augmenting organ wafers were placed into one or more region of the organ, e.g., a kidney. The surface of the recipient kidney was exposed. Renal vessels were clamped temporarily with vascular clamp in order to minimize bleeding. An incision was made on the kidney capsule for accessing the renal parenchyma. The capsule should be carefully pushed away from the parenchyma. The kidney parenchymal tissue similar in size and shape of the renal tissue matrix was removed without disrupting the collecting system during the removal. The cell-seeded renal tissue matrix was placed in the renal parenchyma and the kidney capsule was sutured over the implanted renal tissue matrix. The vascular clamp was removed for recirculation. Hemostasis was achieved by a gentle compression over the implants for a few minutes. After hemostatis, the wound was closed. Following implantation, the growth and development of the cells in the kidney augmenting organ structures was examined. A photograph of renal biomatrices one week after implantation shows that the cells are cell viable and test positive with a lipophilic red fluorescent tracer, carbocyanine at X100 magnification (Photograph not shown). Four weeks after implantation, the formation of tubular and glomerular-like structures is seen (H&E x200, photographs not shown). The development of these tubular structures continues at week 8 post-implantation (photograph not shown).

### Aspects of the present invention:

[1] A kidney function augmenting construct comprising a three-dimensional biomatrix formed by seeding a matrix material with at least one population of cultured cells, such that the cells attach to the matrix and produce a tissue layer capable of augmenting native kidney function.
[2] The construct of [1], wherein at least one population of cells is a population of renal cells and the tissue layer differentiates into a nephron structure, or a part of a nephron structure.
[3] The construct of [1] or [2], wherein the construct is formed by seeding cells onto a matrix material wherein the greatest dimension of the matrix material is less than 50 millimeters.
[4] The construct of any of [1] to [3], wherein the matrix material is decellularized tissue, a hydrogel, or a polymer.
[5] The construct of any [1] to [4], wherein the matrix is a substantially flat structure having a ratio of its greatest dimension to its thickness of greater than 5:1.
[6] The construct of any [1] to [5], further comprising seeding said matrix material with a population of endothelial cells, such that the endothelial cells attach to the matrix to produce an endothelial tissue layer comprising a vascular system, wherein the endothelial cell population is seeded prior to seeding said at least one population of cells.
[7] A method for augmenting the native function of a kidney comprising:
   a) seeding at least one population of cultured cells on or into a matrix material, such that cells attach to the matrix material;
   b) culturing the cells in the matrix material to produce a tissue layer capable of differentiating into an artificial kidney construct, thereby producing a three-dimensional biomatrix; and
   c) implanting the three dimensional biomatrix into at least one target site in the kidney, such that the tissue layer of the three-dimensional biomatrix differentiates to provide a gain of native function to the kidney, thereby augmenting native kidney function at the target site.
[8] A method for augmenting the native function of a kidney comprising:
   a) forming a plurality of three-dimensional biomatrices by seeding a plurality of matrix materials with at least one population of cultured cells;
   b) culturing the cells to produce a tissue layer capable of differentiating; and
   c) implanting the plurality of three-dimensional biomatrices into multiple target sites in the kidney, such that the plurality of three-dimensional biomatrices differentiate to provide a gain of native function to the kidney thereby augmenting native kidney function at the multiple target sites.
[9] The method of [7] or [8], further comprising seeding said matrix material(s) with a population of endothelial cells, such that the endothelial cells attach to the matrix to produce an endothelial tissue layer comprising a vascular system, wherein the endothelial cell population is seeded prior to seeding said at least one population of cells.
[10] The method of any of [7] to [9], wherein the greatest dimension of the matrix is less than 50 millimeters.
[11] The method of any of [7] to [10], wherein the matrix is a substantially flat structure having a ratio of its greatest dimension to its thickness of greater than 5:1.
[12] The method of any of [7] to [11], wherein the matrix is decellularized tissue, a hydrogel, or a polymer.
[13] The method of any of [7] to [12], wherein the at least one population of cells is a population of renal cells, said renal cells producing a tissue layer that differentiates into a nephron structure, or a part of a nephron structure.
[14] The method of [13], wherein the renal cells are an isolated or a mixed population of cells selected from the group consisting of glomeruli cells, proximal tubule cells, distal tubule cells, loop of Henle cells, and collecting duct cells.
[15] The method of [13], wherein the nephron structure consists of the glomerulus, distal tubules, proximal tubules, loop of Henle and collecting ducts.
[16] The method of [13], wherein the part of the nephron structure comprises at least one renal structure selected from the group consisting of the glomerulus, distal tubules, proximal tubules, loop of Henle and collecting ducts.

## Claims

1. A construct for use in augmenting native kidney function in a subject, comprising a three-dimensional biomatrix formed by seeding a polymeric mini-matrix material with at least one population of renal cells such that the cells attach to the matrix material wherein, upon contact of the biomatrix with host tissue at a target site in the subject's native kidney, the kidney function is augmented.

2. The construct for use according to claim 1, wherein the mini-matrix material is a hydrogel.

3. The construct for use according to claim 1, wherein the greatest dimension of the mini-matrix material is less than 50 millimeters, preferably less than 25 millimeters, more preferably less than 10 millimeters, more preferably 2-5 millimeters, and most preferably 2-3 millimeters.

4. The construct for use according to claim 1, wherein the mini-matrix material is 0.01 mm³ to 30 mm³ in volume.

5. The construct for use according to claim 1, wherein the matrix is a substantially flat structure having a ratio of its greatest dimension to its thickness of greater than 5 :1.

6. The construct for use according to claim 1, wherein the polymeric matrix comprises a polymer selected from the group consisting of: collagen, poly (alpha esters), poly (lactic acid), poly (glycolic acid), polyorthoesters, polyanhydrides, cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyestercarbonate, polyether, polyetheretherketone, polyetherimide, polyetherketone, polyethersulfone, polyethylene, polyfluoroolefin, polyimide, polyolefin, polyoxadiazole, polyphenylene oxide, polyphenylene, sulfide, polypropylene, polystyrene, polysulfide, polysulfone, polytetrafluoroethylene, polythioether, polytriazole, polyurethane, polyvinylidene fluoride, regenerated cellulose, ethyl glycol acetate, and urea-formaldehyde.

7. A plurality of the constructs for use according to claim 1 for use in implanting the plurality of constructs into multiple target sites in the native kidney, thereby augmenting kidney function at multiple target sites in the native kidney.

8. The construct for use according to claim 1, wherein the augmented kidney function is glomerular filtration.

9. The construct for use according to claim 1, wherein the augmented kidney function is tubular reabsorption.

## Patentansprüche

1. Konstrukt zur Verwendung in der Verbesserung der natürlichen Nierenfunktion in einem Subjekt, mit einer dreidimensionalen Biomatrix, die durch Einpflanzen eines Polymer-Minimatrixmaterials mit wenigstens einer Nierenzellen-Population gebildet ist, so dass die Zellen an dem Matrixmaterial anhaften, wobei bei Kontakt der Biomatrix mit Wirtsgewebe an einem Zielort in der natürlichen Niere des Subjekts die Nierenfunktion verbessert wird.

2. Konstrukt zur Verwendung gemäß Anspruch 1, wobei das Minimatrixmaterial ein Hydrogel ist.

3. Konstrukt zur Verwendung gemäß Anspruch 1, wobei die größte Abmessung des Minimatrixmaterials weniger als 50 mm, vorzugsweise weniger als 25 mm, besonders bevorzugt weniger als 10 mm, besonders bevorzugt 2-5 mm, und am meisten bevorzugt 2-3 mm beträgt.

4. Konstrukt zur Verwendung gemäß Anspruch 1, wobei die Volumen-Größe des Minimatrixmaterials 0,01 mm³ bis 30 mm³ beträgt.

5. Konstrukt zur Verwendung gemäß Anspruch 1, wobei die Matrix eine im Wesentlichen flache Struktur ist, wobei das Verhältnis von ihrer größter Abmessung zu ihrer Dicke größer als 5:1 ist.

6. Konstrukt zur Verwendung gemäß Anspruch 1, wobei die Polymer-Matrix ein Polymer aufweist, das ausgewählt ist aus der Gruppe bestehend aus: Kollagen, Poly(Alpha-Estern), Poly(Milchsäure), Poly(Glykolsäure), Polyorthoestern, Polyanhydriden, Celluloseether, Cellulose, Celluloseester, fluoriertes Polyethylen, Phenol, Poly-4-Methylpenten, Polyacrylnitril, Polyamid, Polyamidimid, Polyacrylat, Polybenzoxazol, Polycarbonat, Polycyanoarylether, Polyestercarbonat, Polyether, Polyethertherketon, Polyetherimid, Polyetherketon, Polyethersulfon, Polyethylen, Polyfluorolefin, Polyimid, Polyolefin, Polyoxadiazol, Polyphenylenoxid, Polyphenylen, Sulfid, Polypropylen, Polystyren, Polysulfid, Polysulfon, Polytetrafluorethylen, Polythioether, Polytriazol, Polyurethan, Polyvinylidenfluorid, regenerierte Cellulose, Ethylglycolacetat und Harnstoff-Formaldehyd.

7. Mehrere der Konstrukte zur Verwendung gemäß Anspruch 1 zur Verwendung bei der Implantierung der mehreren Konstrukte an mehreren Zielorten in der natürlichen Niere, wodurch die Nierenfunktion an mehreren Zielorte in der natürlichen Niere verbessert wird.

8. Konstrukt zur Verwendung gemäß Anspruch 1, wobei die verbesserte Nierenfunktion Glomerulus-Filtration ist.

9. Konstrukt zur Verwendung gemäß Anspruch 1, wobei die verbesserte Nierenfunktion Tubulus-Reabsorption ist.

## Revendications

1. Construction pour son utilisation dans l'augmentation de la fonction rénale native d'un sujet, comprenant une matrice biologique tridimensionnelle formée en ensemençant un matériau polymérique de mini-matrice avec au moins une population de cellules rénales, de sorte que les cellules s'attachent au matériau de la matrice, dans laquelle, lors du contact de la matrice biologique avec le tissu hôte en un site cible du rein natif du sujet, la fonction rénale est augmentée.

2. Construction pour son utilisation selon la revendication 1, dans laquelle le matériau de la mini-matrice est un hydrogel.

3. Construction pour son utilisation selon la revendication 1, dans laquelle la plus grande dimension du matériau de la mini-matrice est inférieure à 50 millimètres, de préférence inférieure à 25 millimètres, plus préférentiellement inférieure à 10 millimètres, plus préférentiellement 2 à 5 millimètres et le plus préférentiellement 2 à 3 millimètres.

4. Construction pour son utilisation selon la revendication 1, dans laquelle le matériau de la mini-matrice a un volume de 0,01 mm³ à 30 mm³.

5. Construction pour utilisation selon la revendication 1, dans laquelle la matrice est une structure sensiblement plate dont le rapport de la plus grande dimension sur l'épaisseur est supérieur à 5:1.

6. Construction pour son utilisation selon la revendication 1, dans laquelle la matrice polymérique comprend un polymère choisi dans le groupe constitué de : collagène, poly(esters alpha), poly(acide lactique), poly(acide glycolique), polyorthoesters, polyanhydrides, éther de cellulose, cellulose, ester cellulosique, polyéthylène fluoré, phénolique, poly-4-méthylpentène, polyacrylonitrile, polyamide, polyamide-imide, polyacrylate, polybenzoxazole, polycarbonate, polycyanoaryléther, polyestercarbonate, polyéther, polyétheréthercétone, polyétherimide, polyéthercétone, polyéthersulfone, polyéthylène, polyflurooléfine, polyimide, polyoléfine, polyoxadiazole, oxyde de polyphénylène, polyphénylène, sulfure, polypropylène, polystyrène, polysulfure, polysulfone, polytétrafluoroéthylène, polythioéther, polytriazole, polyuréthane, fluorure de polyvinylidène, cellulose régénérée, acétate d'éthylglycol et urée-formaldéhyde.

7. Pluralité de constructions pour son utilisation selon la revendication 1, afin d'implanter une pluralité de constructions en des sites cibles multiples dans le rein natif, augmentant ainsi la fonction rénale en des sites cibles multiples dans le rein natif.

8. Construction pour utilisation selon la revendication 1, dans laquelle la fonction rénale augmentée est la filtration glomérulaire.

9. Construction pour utilisation selon la revendication 1, dans laquelle la fonction rénale augmentée est la réabsorption tubulaire.
